# EUROPÄISCHE PATENTSCHRIFT

(11) **EP 1 263 705 B1**
(45) Veröffentlichungstag und Bekanntmachung des Hinweises auf die Patenterteilung: **19.11.2003**
(21) Anmeldenummer: 01923675.1
(22) Anmeldetag: 16.03.2001
(51) Int. Cl.: C07C 46/06, C07C 50/14

(54) **VERFAHREN ZUR HERSTELLUNG VON 1,1'-BINAPHTALINYLIDEN-4,4'-DIONEN**
METHOD FOR PRODUCING 1,1' BINAPHTHALENYLIDENE-4,4'-DIONES
PROCEDE DE PRODUCTION DE 1,1'-BINAPHTALINYLIDENE-4,4'-DIONES

(30) Priorität: 17.03.2000 EP 00105700
(43) Veröffentlichungstag der Anmeldung: 11.12.2002
(73) Patentinhaber: Lonza AG, 4052 Basel (CH)
(72) Erfinder: HAUCK, Michael, CH-3904 Naters (CH); HEVELING, Josef, Ifafi 0260, Hartbeespoort (ZA); WELLIG, Alain, CH-3986 Ried-Mörel (CH)
(86) Internationale Anmeldenummer: EP0103045
(87) Internationale Veröffentlichungsnummer: WO01068579

(56) Entgegenhaltungen:
- EP-A- 0 812 816
- KRAL, ANDREAS ET AL: "4,4'-Binaphthylidendione - Farbe und Struktur" Z. NATURFORSCH., B: CHEM. SCI. (1993), 48(10), 1401-7 , XP002171013
- ORR S F D ET AL: "The oxidation of 2-naphthylamine with benzoyl peroxide" JOURNAL OF THE CHEMICAL SOCIETY., 1956, Seiten 1337-1344, XP002171014 CHEMICAL SOCIETY. LETCHWORTH., GB

## Beschreibung

Die vorliegende Erfindung betrifft ein Verfahren zur Herstellung von 1,1 '-Binaphthalinyliden-4,4'-dionen.

1,1'-Binaphthalinyliden-4,4'-dione können beispielsweise durch oxidative Kupplung von 1-Naphtholen in Gegenwart von Silberoxid hergestellt werden. Ein entsprechendes Herstellungsverfahren wird von A. Kral et al. in *Z. Naturforsch. B* **1993**, *48*, 1401-1407 beschrieben.

Die bekannten Verfahren sind keine katalytischen Verfahren. Dies führt zu einem hohen Verbrauch an meist schwermetallhaltigen Oxidantien, die entweder regeneriert oder mit zusätzlichen Kosten umweltgerecht entsorgt werden müssen.

Aufgabe der vorliegenden Erfindung war es daher, ein Verfahren zu finden, das die oben genannten Nachteile nicht aufweist.

Erfindungsgemäss wird diese Aufgabe durch das Verfahren nach Patentanspruch 1 gelöst.

Es wurde nun gefunden, dass 1,1'-Binaphthalinyliden-4,4'-dione der allgemeinen Formel 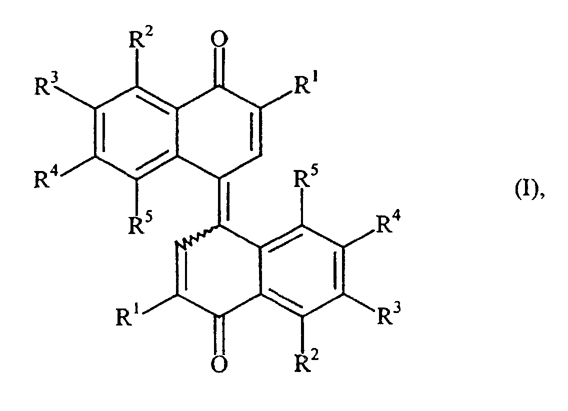 worin
- R¹: C₁₋₆-Alkyl, C₁₋₆-Alkoxy, Phenyl, substituiertes Phenyl, Benzyl oder Benzyloxy;
- R², R³, R⁴ und R⁵: unabhängig voneinander Wasserstoff, C₁₋₆-Alkyl, C₁₋₆-Alkoxy, Phenyl, substituiertes Phenyl, Benzyl oder Benzyloxy bedeuten,
durch oxidative Kupplung von Naphtholen der allgemeinen Formel 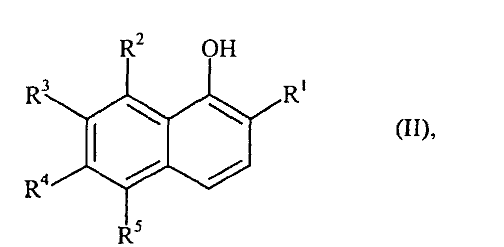 worin R¹, R², R³, R⁴ und R⁵ die oben angegebene Bedeutung haben,
in Gegenwart eines Peroxids sowie eines Edelmetallkatalysators hergestellt werden können.

Der Rest R¹ ist vorzugsweise Methyl. Die Reste R², R³, R⁴ und R⁵ sind vorzugsweise Wasserstoff.

Die Verbindungen der Formel 1 können sowohl in der (*E*)-Form als auch in der (*Z*)-Form auftreten. Bevorzugt ist die (*E*)-Form.

Unter C₁₋₆-Alkyl sind hier und im folgenden alle linearen oder verzweigten Alkylgruppen mit 1-6 Kohlenstoffatomen zu verstehen, wie beispielsweise Methyl, Ethyl, Propyl, Isopropyl, Butyl, Isobutyl, *sec*-Butyl, *tert-*Butyl, Pentyl, Isopentyl, *tert*-Pentyl, Neopentyl, Hexyl oder Isohexyl.

Unter C₁₋₆-Alkoxy sind hier und im folgenden alle linearen oder verzweigten Alkoxygruppen mit 1-6 Kohlenstoffatomen zu verstehen, wie beispielsweise Methoxy, Ethoxy, Propoxy, Isopropoxy, Butoxy, Isobutoxy, *sec*-Butoxy, *tert*-Butoxy, Pentyloxy, Isopentyloxy, *tert*-Pentyloxy, Neopentyloxy, Hexyloxy oder Isohexyloxy.

Der Phenylrest kann auch einen oder mehrere gleiche oder verschiedene Substituenten in den ortho-, meta-, oder para-Stellungen tragen. Als Substituenten kommen beispielsweise Halogene wie Fluor, Chlor, Brom oder Iod, C₁₋₆-Alkyl, halogeniertes C₁₋₆-Alkyl wie beispielsweise Trifluormethyl oder C₁₋₆-Alkoxy in Frage. Substituierte Phenylreste sind beispielsweise Methylphenyl, Dimethylphenyl, Ethylphenyl, Propylphenyl, Methoxyphenyl, Ethoxyphenyl, Propoxyphenyl und dergleichen.

Die Naphthole der Formel II sind bekannte Verbindungen oder analog zu bekannten Verbindungen herstellbar. 2-Alkyl-1-naphthole sind leicht durch Reduktion der entsprechenden Arylalkylketone oder durch Alkylierung zugänglich. Die Synthese von 2-Alkoxy-1-naphtholen kann beispielsweise ausgehend von den entsprechenden 2-Alkoxynaphthalin-1-carbaldehyden erfolgen, wie in der oben zitierten Literatur beschrieben.

Ein bevorzugtes Naphthol ist 2-Methyl-1-naphthol.

Unter Peroxiden sind sowohl organische als auch anorganische Peroxide zu verstehen. Beispiele geeigneter Peroxide sind Wasserstoffperoxid, Perbenzoesäure oder Peressigsäure. Bevorzugt ist Wasserstoffperoxid, vorteilhafterweise als wässrige 10-30%ige Lösung. Besonders bevorzugt ist eine 30%ige wässrige Lösung von Wasserstoffperoxid.

Als Edelmetallkatalysatoren eignen sich insbesondere Platin-, Rhodium- oder Ruthenium-Katalysatoren. Bevorzugt ist ein Platin-Katalysator.

Vorteilhaft werden die Edelmetallkatalysatoren in Kombination mit anderen Metallen wie beispielsweise Bismut, Blei oder Cer eingesetzt. Bevorzugt ist hier eine Kombination von Platin und Bismut.

Die Katalysatoren können trägerfrei oder auf einem geeigneten Trägermaterial aufgebracht, eingesetzt werden. Vorzugsweise werden Trägerkatalysatoren eingesetzt. Als Träger sind alle gebräuchlichen Trägermaterialien geeignet wie z. B. Aktivkohle, Aluminiumoxid, Siliciumoxid, Silicium-Aluminiumoxid, Siliciumcarbid, Titandioxid, Magnesiumoxid oder Zeolithe. Besonders bevorzugt ist Aktivkohle.

Die Trägermaterialien enthalten vorteilhaft 0,1-30 Gew.%, vorzugsweise 0,5-10 Gew.% an Metall.

Besonders bevorzugt ist ein Platin/Bismut-Katalysator auf Aktivkohle, beispielsweise mit 5% Pt und 5% Bi.

Diese Katalysatoren sind im Handel erhältlich, z. B. bei Degussa oder Heraeus.

Das erfindungsgemässe Verfahren wird vorteilhaft bei einer Temperatur von 20-120 °C, vorzugsweise bei 50-100 °C durchgeführt.

Das erfindungsgemässe Verfahren wird vorteilhaft in einem geeigneten Lösungsmittel durchgeführt. Als Lösungsmittel eignen sich insbesondere niedere Alkohole wie Methanol, Ethanol, Propanol, Isopropanol oder Gemische von Alkoholen mit Essigsäure.

Die Verbindungen der Formel 1 absorbieren sichtbares Licht im Bereich von 480 nm bis 560 nm und eignen sich deshalb als Farbstoffe, wie in der oben zitierten Literatur beschrieben.

Das folgende Beispiel verdeutlicht die Durchführung des erfindungsgemässen Verfahrens, ohne dass darin eine Einschränkung zu sehen ist.

### Beispiel

### 3,3'-Dimethyl-1,1'-binaphthalinyliden-4,4'-dion

5 g ( 31,6 mmol) 2-Methyl-1-naphthol, 50 g Methanol und 0,4 g eines Katalysators mit 5% Pt und 5% Bi auf Aktivkohle (Degussa) wurden in einem 100 ml Dreihalskolben vorgelegt. Das Reaktionsgemisch wurde unter Rühren im Ölbad auf 60 °C aufgeheizt. Innerhalb einer Stunde wurden 8 g 30%ige wässrige H₂O₂-Lösung in die Lösung getropft (70,6 mmol H₂O₂). Während der Reaktion fiel ein roter Farbstoff aus. Um die Rührbarkeit zu gewährleisten, wurden 25 min nach Beginn des Zutropfens weitere 15 ml Methanol zugesetzt. Nach weiteren 5 min wurden 10 ml Essigsäure (konz.) zugegeben. Bei Ende der H₂O₂-Zugabe lag eine intensiv rot gefärbte Suspension vor. Man liess abkühlen, anschliessend wurde über eine G4-Nutsche filtriert. Der Filterkuchen wurde mit 20 ml Methanol gewaschen. Das eingedampfte Filtrat ergab 0,8 g eines dunkelroten Harzes (Fraktion 1). Der Filterkuchen wurde nacheinander mit Aceton, Methylenchlorid und dann wieder mit Aceton aufgeschlämmt und jeweils filtriert. Die gesammelten Filtrate wurden zur Trockne eingeengt; man erhielt 0,8 g eines dunkelroten Feststoffes (Fraktion 2). Der Filterkuchen wurde dann in 1,5 l heissem Aceton gelöst. Die Lösung wurde über eine G4-Nutsche, in die man zusätzlich eine 2 cm dicke Celiteschicht gegeben hatte, vom Katalysator befreit. Die Filterschicht wurde mit 40 ml heissem Aceton nachgewaschen. Beim Einengen der Acetonlösung (60 °C/450 mbar) auf ein Volumen von ca. 200 ml kristallisierte ein roter Feststoff aus. Man versetzte das Gemisch mit 150 ml Methanol und engte weiter auf ein Gesamtvolumen von ca. 150 ml ein. Dann liess man auf 25 °C abkühlen und filtrierte. Nach dem Trocknen (50 °C/25 mbar) erhielt man Fraktion 3 (3,5 g). Durch Einengen des Filtrats erhielt man eine weitere Fraktion 4 (0,3 g).
Der rote Feststoff wurde durch Elementaranalyse, UV, IR und MS, ergänzt durch ¹H NMRund ¹³C NMR-Messungen charakterisiert und als 3,3'-Dimethyl-1,1'-binaphthalinyliden-4,4 'dion identifiziert.
Die Reinheit der nach oben beschriebenem Vorgehen erhaltenen Fraktion 3 wurde mittels NMR auf ca. 80% geschätzt.
Insgesamt wurden 5,4 g Feststoff erhalten. Legt man einen Gehalt von 80% zugrunde, beträgt die Ausbeute 87,5%.

## Patentansprüche

1. Verfahren zur Herstellung von 1,1'-Binaphthalinyliden-4,4'-dionen der allgemeinen Formel 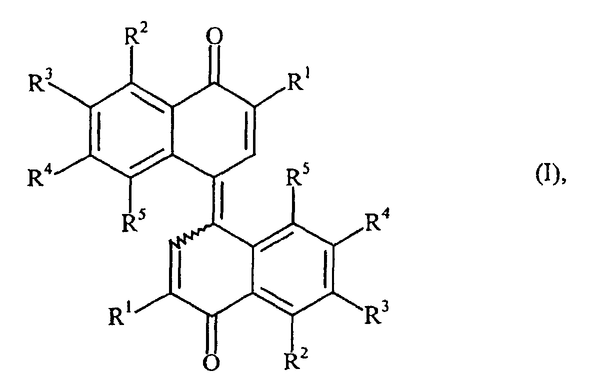 worin
R¹ C₁₋₆-Alkyl, C₁₋₆-Alkoxy, Phenyl, substituiertes Phenyl, Benzyl oder Benzyloxy;
R², R³, R⁴ und R⁵ unabhängig voneinander Wasserstoff, C₁₋₆-Alkyl, C₁₋₆-Alkoxy, Phenyl, substituiertes Phenyl, Benzyl oder Benzyloxy bedeuten,
durch oxidative Kupplung von Naphtholen der allgemeinen Formel 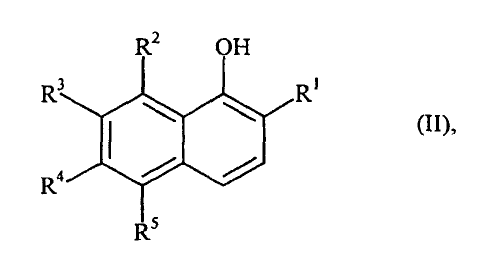 worin R¹, R², R³, R⁴ und R⁵ die oben angegebene Bedeutung haben,
**dadurch gekennzeichnet, dass** die oxidative Kupplung in Gegenwart eines Peroxids sowie eines Edelmetallkatalysators erfolgt.

2. Verfahren nach Anspruch 1, worin R¹ Methyl ist und R², R³, R⁴ und R⁵ Wasserstoff bedeuten.

3. Verfahren nach Anspruch 1 oder 2, **dadurch gekennzeichnet, dass** als Peroxid Wasserstoffperoxid, insbesondere als wässrige 10-30%ige Lösung, eingesetzt wird.

4. Verfahren nach einem der Ansprüche 1 bis 3, **dadurch gekennzeichnet, dass** als Edelmetallkatalysator ein Platin-, Rhodium- oder Ruthenium-Katalysator, gegebenenfalls in Kombination mit Bismut, Blei oder Cer eingesetzt wird.

5. Verfahren nach Anspruch 4, **dadurch gekennzeichnet, dass** der Katalysator auf einem Trägermaterial aufgebracht ist.

6. Verfahren nach Anspruch 5, **dadurch gekennzeichnet, dass** der Katalysator ein Pt/Bi-Katalysator auf Aktivkohleträger ist.

7. Verfahren nach einem der Ansprüche 1 bis 6, **dadurch gekennzeichnet, dass** die oxidative Kupplung bei einer Temperatur von 20-120 °C, vorzugsweise bei 50-100 °C durchgeführt wird.

8. Verfahren nach einem der Ansprüche 1 bis 7, **dadurch gekennzeichnet, dass** die oxidative Kupplung in einem Alkohol oder einem Alkohol/Essigsäure-Gemisch durchgeführt wird.

## Claims

1. A process for preparing 1,1'-binaphthalenylidene-4,4'-diones of the general formula 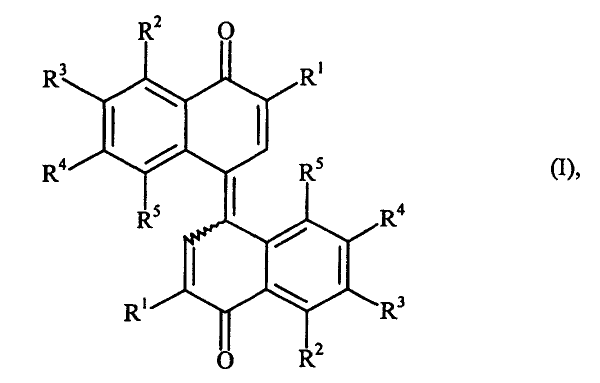 wherein
R¹ is C₁₋₆-alkyl, C₁₋₆-alkoxy, phenyl, substituted phenyl, benzyl or benzyloxy;
R², R³, R⁴ and R⁵ are each independently hydrogen, C₁₋₆-alkyl, C₁₋₆-alkoxy, phenyl, substituted phenyl, benzyl or benzyloxy,
by oxidative coupling of naphthols of the general formula 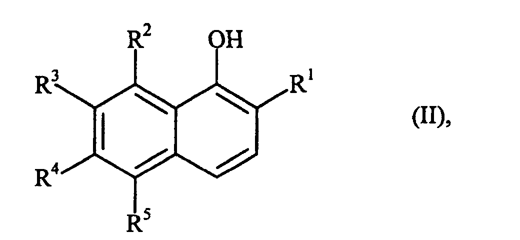 where R¹, R², R³, R⁴ and R⁵ are each as defined above,
**characterized in that** the oxidative coupling is effected in the presence of a peroxide and also of a noble metal catalyst.

2. The process as claimed in claim 1, where R¹ is methyl and R², R³, R⁴ and R⁵ are each hydrogen.

3. The process as claimed in claim 1 or 2, **characterized in that** the peroxide used is hydrogen peroxide, in particular as an aqueous 10-30% solution.

4. The process as claimed in any of claims 1 to 3, **characterized in that** the noble metal catalyst used is a platinum, rhodium or ruthenium catalyst, optionally in combination with bismuth, lead or cerium.

5. The process as claimed in claim 4, **characterized in that** the catalyst is applied to a support material.

6. The process as claimed in claim 5, **characterized in that** the catalyst is a Pt/Bi catalyst on an activated carbon support.

7. The process as claimed in any of claims 1 to 6, **characterized in that** the oxidative coupling is carried out at a temperature of 20-120 °C, preferably 50-100 °C.

8. The process as claimed in any of claims 1 to 7, **characterized in that** the oxidative coupling is carried out in an alcohol or an alcohol/acetic acid mixture.

## Revendications

1. Procédé de préparation de 1,1'-binaphtalinylidèn-4,4'-diones de la formule générale 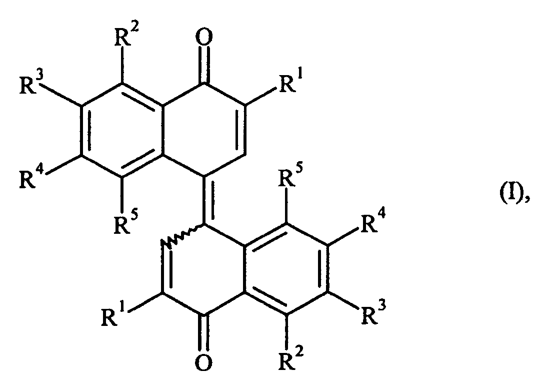 où
R¹ désigne un résidu C₁₋₆ alkyle, C₁₋₆ alkoxy, phényle, phényle substitué, benzyle ou benzyloxy ;
R², R³, R⁴ et R⁵ représentent, indépendamment les uns des autres, l'hydrogène, des résidus C₁₋₆ alkyle, C₁₋₆ alkoxy, phényle, phényle substitué, benzyle ou benzyloxy,
par couplage oxydant de naphtols de la formule générale 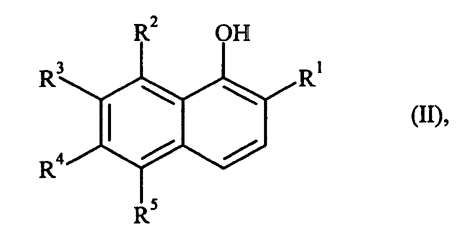 où R¹, R², R³, R⁴ et R⁵ ont la signification indiquée ci-dessus,
**caractérisé en ce que** le couplage oxydant se fait en présence d'un peroxyde ainsi que d'un catalyseur à métaux précieux.

2. Procédé selon la revendication 1, où R¹ est un résidu méthyle et R², R³, R⁴ et R⁵ signifient l'hydrogène.

3. Procédé selon la revendication 1 ou la revendication 2, **caractérisé en ce que** l'on utilise, en tant que peroxyde, le peroxyde d'hydrogène, en particulier, en tant que solution aqueuse à raison de 10-30%.

4. Procédé selon l'une quelconque des revendications 1 à 3, **caractérisé en ce que** l'on utilise, en tant que catalyseur à métaux précieux, un catalyseur au platine, au rhodium ou au ruthénium, le cas échéant, en combinaison à du bismuth, du plomb ou du cérium.

5. Procédé selon la revendication 4, **caractérisé en ce que** le catalyseur est appliqué sur un matériau de support.

6. Procédé selon la revendication 5, **caractérisé en ce que** le catalyseur est un catalyseur Pt/Bi sur un support de charbon actif.

7. Procédé selon l'une quelconque des revendications 1 à 6, **caractérisé en ce que** le couplage oxydant est effectué à une température de 20-120 °C, de préférence, de 50-100 °C.

8. Procédé selon l'une quelconque des revendications 1 à 7, **caractérisé en ce que** le couplage oxydant est effectué dans un alcool ou dans un mélange alcool/acide acétique.
